# EUROPÄISCHE PATENTSCHRIFT

(11) **EP 1 576 186 B1**
(45) Veröffentlichungstag und Bekanntmachung des Hinweises auf die Patenterteilung: **04.05.2011**
(21) Anmeldenummer: 03775367.0
(22) Anmeldetag: 18.11.2003
(51) Int. Cl.: C12Q 1/68, C12N 1/00

(54) **VERFAHREN ZUR GENERIERUNG EINES GENTECHNISCH VERÄNDERTEN ORGANISMUS FÜR DAS WIRKSUBSTANZSCREENING**
METHOD FOR GENERATING A GENETICALLY MODIFIED ORGANISM FOR SCREENING ACTIVE SUBSTANCES
PROCEDE POUR GENERER UN ORGANISME GENETIQUEMENT MODIFIE SERVANT AU CRIBLAGE D'UNE SUBSTANCE ACTIVE

(30) Priorität: 17.12.2002 DE 10258885
(43) Veröffentlichungstag der Anmeldung: 21.09.2005
(73) Patentinhaber: Sanofi-Aventis Deutschland GmbH, 65929 Frankfurt am Main (DE)
(72) Erfinder: KLEBL, Bert, 82294 Günzlhofen (DE); STADLER, Anja, 82256 Fürstenfeldbruck (DE); SÖLLNER, Rosemarie, 65926 Frankfurt am Main (DE); LEBERER, Ekkehard, 82110 Germering (DE); NITSCHE, Almut, D-65926 Frankfurt am Main (DE)
(86) Internationale Anmeldenummer: PCT/EP2003/012870
(87) Internationale Veröffentlichungsnummer: WO 2004/055206

(56) Entgegenhaltungen:
- WO-A-02/063029
- WO-A-03/016568
- TUGENDREICH STUART ET AL: "A streamlined process to phenotypically profile heterologous cDNAs in parallel using yeast cell-based assays" GENOME RESEARCH, Bd. 11, Nr. 11, November 2001 (2001-11), Seiten 1899-1912, XP002272319 ISSN: 1088-9051
- CAUMONT ANNE B ET AL: "Expression of functional HIV-1 integrase in the yeast Saccharomyces cerevisiae leads to the emergence of a lethal phenotype: Potential use for inhibitor screening" CURRENT GENETICS, Bd. 29, Nr. 6, 1996, Seiten 503-510, XP002272320 ISSN: 0172-8083
- PAUSCH M H: "G-protein-coupled receptors in Saccharomyces cerevisiae: high-throughput screening assays for drug discovery" TRENDS IN BIOTECHNOLOGY, ELSEVIER PUBLICATIONS, CAMBRIDGE, GB, Bd. 15, Nr. 12, 1. Dezember 1997 (1997-12-01), Seiten 487-494, XP004097426 ISSN: 0167-7799
- ATIENZA J M ET AL: "Yeast Model System for Study of Mammalian Phosphodiesterases" METHODS: A COMPANION TO METHODS IN ENZYMOLOGY, ACADEMIC PRESS INC., NEW YORK, NY, US, Bd. 14, Nr. 1, Januar 1998 (1998-01), Seiten 35-42, XP004466609 ISSN: 1046-2023

## Beschreibung

Es ist bekannt, zum Wirksubstanzscreening, gentechnisch veränderte Hefen einzusetzen, die das Zielprotein, welches durch die zu testende Substanz inhibiert werden soll, heterolog exprimieren. Heterologe Expression bedeutet im im Rahmen dieser Erfindung die Expression eines dem Organismus fremden Gens oder die Expression eines dem Organismus eigenen Gens mit verändertem Expressionsmuster, insbesondere verstärkter oder verminderter Expression, und/oder zeitlich und/oder räumlich (z.B. andere Kompartimente, bei höheren Organismen z.B. andere Gewebe) veränderter Expression. Im einfachsten Fall führt die heterologe Expression zu einem detektierbaren, veränderten Phänotyp, meist einer Wachstumsinhibierung der Hefe. Wachstumsinhibierung bedeutet im Rahmen der vorliegenden Erfindung eine verminderte Proliferationsrate und/oder ein vermindertes Größenwachstum und schließlt auch den Zelltod (apoptotisch oder nekrotisch) ein. Die Art der auftretenden Wachstumsinhibierung hängt auch vom Organismus ab, so ist bei Hefen eher ein Proliferationsarrest oder eine Lyse zu beobachten, bei eukaryontischen Zellen, die ursprünglich aus Vielzellern stammen, ist dagegen z.T. auch Apoptose zu beobachten. Führt die heterologe Expression zu einer von aussen wahrnehmbaren Veränderung von Verhalten und/oder Morphologie des Organismus (also einem veränderten Phänotyp), so kann der gentechnisch veränderte Organismus einfach für das Wirksubstanzscreening eingesetzt werden, wobei die Wirksamkeit der getesteten Substanzen anhand ihrer Fähigkeit, den Phänotyp (z.B. die Wachstumsinhibierung) aufzuheben oder zu vermindern, feststellbar ist. Dies erfolgt beim Beispiel Hefesystem mit Wachstumsinhibierung als verändertem Phänotyp vorzugsweise durch einfache Wachstumsassays, die sich auch für das Hochdurchsatz-Screening (HTS) eignen. Als veränderter Phänotyp wird jede von aussen wahrnehmbare Veränderung des gentechnisch veränderten Organismus (Gestalt, Größe, etc.) oder seines Verhaltens (Wachstum, Zellteilungsrate, etc.) gegenüber dem des gentechnisch nicht veränderten bzw. Dem das oder die heterologen Proteine oder - Fragmente nicht exprimierenden Organismus bezeichnet. Phänotypisierung bezeichnet somit die Herbeiführung einer solchen Veränderung. Dieses Verfahren des Standes der Technik weist jedoch den Nachteil auf, dass nur ein geringer Teil heterolog exprimierter Gene einen für das Wirksubstanzscreening nutzbaren Phänotyp des gentechnisch veränderten Organismus hervorbringt. So wird vermutet, daß beispielsweise nur ca. 20-30 % aller heterolog exprimierten Kinasen eine für das Wirksubstanzscreening nutzbare Wachstumshemmung in der Hefe verursachen. Bei den übrigen 70-80% ist die Wachstumshemmung so gering, dass sie für das Screening nicht nutzbar ist (zu geringer Unterschied im Vergleich zur Kontrolle führt zu einem hohen Hintergrund und somit zu einer zu hohen Zahl an falsch Positiven), oder sie ist gar nicht vorhanden.

Es besteht daher Bedarf an einem Verfahren zur Generierung eines gentechnischen Organismus für das Wirksubstanzscreening, welcher die Nachteile des Standes der Technik nicht aufweist und insbesondere dafür geeignet ist, auch solche heterolog exprimierten Gene dem Wirksubstanzscreening zuzuführen, welche in dem sie heterolog exprimierenden Organismus keinen bzw. keinen für das Screening, insbesondere das HTS, nutzbaren Phänotyp hervorbringen.

Diese Aufgabe wird erfindungsgemäß gelöst durch ein Verfahren zur Generierung eines gentechnisch veränderten Organismus für das Wirksubstanzscreening, bei dem
a) ein Protein oder Proteinfragment In einem eukaryontischen einzelligen Organismus durch gentechnische Veränderung exprimiert wird,
b) das dadurch veränderte Genexpressionsmuster des eukaryontischen einzelligen Organismus mit Hilfe eines DNA- oder Protein-Microarrays analysiert wird,
c) Gene identifiziert werden, deren Expression oder Abschalten die Auswirkungen der Expression des Proteins oder Proteinfragmentes kompensieren, und
d) durch Minderung, Aufhebung oder Verstärkung der Expression dieser kompensatorischen Gene ein Phänotyp in dem gentechnisch veränderten eukaryontischen einzelligen Organismus hervorgerufen wird, wobei der Phänotyp die Wachstumshemmung des eukaryontischen einzelligen Organismus ist, wobei der Organismus eine Hefezelle ist.

Die Erfindung beruht auf der Erkenntnis der Erfinder, dass das Fehlen eines detektierbaren Phänotyps bei Expression der meisten Gene, seien es fremde Gene oder organismuseigene Gene, darauf beruht, dass der gentechnisch veränderte Organismus die Expression eigener Gene als Antwort auf die Expression des exprimierten Proteins oder Proteinfragmentes hoch- oder herunterreguliert (d.h. kompensatorisch differentiell reguliert). Differentiell reguliert bedeutet in diesem Fall, anders reguliert als im nicht gentechnisch veränderten Organismus, bzw. ohne die Expression des exprimierten Proteins oder Proteinfragmentes. Kompensatorisch bedeutet, dass diese Differentielle Genregulation eine Antwort auf die Expression des Proteins oder Proteinfragmentes ist. Solche differentiell regulierten Gene werden in der vorliegenden Erfindung auch als kompensatorische Gene bezeichnet.

Die Erfindung ermöglicht die Entwicklung einer Plattformtechnologie in einem zellulären, im Gegensatz zum einfach biochemischen, Modell, vorzugsweise der Hefe. Mit dem Assaysystem können Inhibitoren beispielsweise aus chemischen Bibliotheken, aus Combichembibliotheken und aus Naturstoffextrakten identifiziert werden. Das Assaysystem kann auf 96-, 384- oder 1536-well Platten oder andere für zelluläre Assays gängige Formate angepasst werden. Das zu wählende Format hängt z.T. auch vom gewählten Organismus ab, die Auswahl liegt dabei im Bereich des fachmännischen Könnens.

Das erfindungsgemäße Verfahren eignet sich insbesondere für Gene bzw. Proteine oder -Fragmente, deren Expression im gewünschten Organismus zu keiner detektierbaren Veränderung des Phänotyps gegenüber dem der gentechnisch unveränderten, bzw. das Protein oder -Fragment nicht exprimierenden Organismus führt. Es können beispielsweise Proteinkinasen ebenso wie andere Genprodukte getestet werden, die eine transkriptionelle Antwort auslösen. Es ist jedoch ebenso bei detektierbar verändertem Phänotyp anwendbar, insbesondere dann, wenn zwar ein veränderter Phänotyp detektierbar ist, aber aus bestimmten Gründen für den Einsatz im Wirksubstanzscreening nicht geeignet oder nicht zweckmäßig ist. Dieser kann durch die Phänotyisierung verstärkt oder so verändert werden. dass er für das Wirkstoff-Screening nutzbar ist. Die Phänotypisierung bezeichnet im Rahmen der vorliegenden Erfindung demnach die Herbeiführung oder

Verstärkung eines vom das oder die Proteine oder -Fragmente nicht exprimierenden bzw. vom nicht gentechnisch veränderten Organismus unterscheidbaren Phänotyps im das oder die Proteine oder-Fragmente exprimierenden, gentechnisch veränderten Organismus. Als Organismen eignen sich vorzugsweise Zellen, hier eukaryontische Zellen ebenso wie prokaryontische, aber auch vielzellige nicht-humane Organismen, die sich für das Wirksubstanzscreening eignen, z.B. Drosophila und vorzugsweise C. Elegans. Als eukaryontische Zellen eignen sich vorzugsweise kultivierte Zellinlen, die ursprünglich aus vielzelligen Organismen gewonnen wurden, z.B. 3T3, CHO, Hela, aber auch andere oder eukaryontische einzellige Organismen, insbesondere Hefen. Unter den Hefen eignen sich wiederum insbesondere solche der Stämme S. cerevisiae oder S. pombe. Dem Fachmann sind geeignete Laborstämme von Hefezellen oder geeignete eukaryontische Zellinien hinlänglich bekannt. Von der vorliegenden Erfindung sind Hefezellen umfasst. Als Proteine und Proteinfragmente kommen grundsätzlich alle die in Frage, deren heterologe Expression im Organismus zu einer Veränderung des Expressionsmusters eigener Gene führt. Vorteilhaft sind alle Proteine und-fragmente, die für die Findung neuer Wirkstoffe von Interesse sind, im Rahmen dieser Erfindung sind besonders bevorzugt Kinasen, Phosphatasen, GPCRs, (insbesondere kleine) GTPasen, Proteasen und Ionenkanäle.

Der Begriff Wirksubstanzscreening umfasst im Rahmen dieser Erfindung jede Art der Suche von Substanzen, die sich auf die Aktivität eines oder mehrerer bestimmter Zielgene und/oder Zielproteine auswirken, unter Einsatz mindestens eines gentechnisch veränderten Organismus. Es kommen dabei grundsätzlich alle Arten von Substanzen in Frage, beispielsweise alle Arten von Naturstoffen (also in der Natur vorkommende Moleküle, insbesondere Biomoleküle) ebenso wie nicht natürlch vorkommende, synthetisch hergestellte Chemikalien und von Naturstoffen, insbesondere biologischen Molekülen abgeleitete Substanzen/ Derivate (z.B. modifizierte Peptide oder Oligonukleotide).

In einem ersten Schritt des Verfahrens der vorliegenden Erfindung wird ein Protein oder Proteinfragment in einer Hefezelle durch gentechnische Veränderung exprimiert.

Die Expression kann die Einschleusung eines fremden Gens aber auch die veränderte Expression eines Organismus-eigenen Gens, z.B. durch die Einschleusung eines entsprechenden Expressionsvektors umfassen. Die dazu notwendige, gentechnische Veränderung kann dabei die Veränderung des Organismengenoms (z.B. durch stabile, ins Genom integrierende Vektoren oder durch verschiedene Arten der Mutagenase) betreffen, episomal sein oder einfach die Einschleusung geeigneter Vektoren umfassen, die zum Verbleib im Organismus der ständigen Selektion mittels eines oder mehrerer Selektionsmarker bedürfen. Die bestgeeignete Art hängt von verschiedenen Faktoren, u.a. auch von der Art des Organismus ab und ist für den zuständigen Fachmann einfach bestimmbar.

Die Expression betrifft dabei mindestens ein Protein oder -fragment, kann aber auch mehrere Proteine oder -fragmente betreffen. Es kann zweckmässig sein, die Expression des Proteins/Fragmentes durch geeignete Methoden (PCR, Northem-, Westemblot etc.) zu verifizieren, bevor das Genexpressionsmuster des gentechnisch veränderten Organismus mit dem ohne die Expression des Proteins verglichen und so analysiert wird. In einem zweiten Schritt des Verfahrens der vorliegenden Erfindung wird das durch die Expression des Proteins oder Proteinfragments veränderte Genexpressionsmuster der Hefezellen analysiert. Die Analyse erfolgt durch geeignete Maßnahmen, die dem Fachmann hinlänglich bekannt sind. Insbesondere eignet sich dazu der Einsatz von Array- oder Chip-Systemen. Im Verfahren der vorliegenden Erfindung erfolgt die Analyse mit Hilfe von DNA- oder Proteinmicroarrays. Durch Vergleich der Expressionsmuster eines Kontroll - Organismus (z.B. eines Wildtyp-Organismus oder eines Organismus, in den lediglich der Leervektor eingeschleust wurde, oder bei induzierbaren Systemen der genetechnisch veränderte Organismus, bei dem die Expression des Gens für das Protein- oder Proteinfragment nicht induziert ist) und des gentechnisch veränderten, das heterologe Gen exprimierenden Organismus werden in einem dritten Schritt Gene identifiziert, deren Expression oder Abschaltung die Auswirkungen der Expression des Proteins oder Proteinfragments kompensieren. Solche Gene, die im Expressionsmuster des gentechnisch veränderten, das heterologe Gen exprimierenden Organismus im Gegensatz zu dem Expressionsmuster des Kontrollorganismus überhaupt / verstärkt *l* vermindert oder gar nicht exprimiert werden, werden somit als kompensatorische Gene erachtet und können für die Phänotypisierung des gentechnisch veränderten Organismus eingesetzt werden.

In einem vierten Schritt des Verfahrens der vorliegenden Erfindung wird durch Minderung, Aufhebung oder Verstärkung der Expression der kompensatorischen Gene ein Phänotyp in der gentechnisch veränderten Hefezelle hervorgerufen, wobei der Phänotyp die Wachstumshemmung der Hefezelle ist. Die als Phänotypisierung bezeichnete Veränderung bezieht sich auf die Herbeiführung oder Verstärkung eines vom Wildtyporganismus unterscheidbaren Phänotyps im gentechnischen veränderten Organismus (oder, bei induzierbaren Systemen, eines Phänotyps, der nur bei Expression des oder der Proteine oder - Fragmente durch den gentechnisch veränderten Organismus ausgebildet wird und im nicht-induziertem Zustand des Organismus, wenn das oder die Proteine oder -Fragmente nicht exprimiert werden, nicht ausgebildet ist), vorzugsweise handelt es sich dabei um einen für die Auswertung in HTS-Wirksubstanzscreening geeigneten Phänotyp. Die Herbeiführung oder Verstärkung kann dabei beispielsweise auf der Minderung oder Aufhebung der Expression eines oder mehrerer kompensatorisch hochregulierter Gene (dies kann z.B. durch genomischen Knock Out einer oder mehrerer der kompensatorisch differentiell regulierten Gene oder durch Mutagenese erfolgen) oder der verstärkten Expression eines oder mehrerer kompensatorisch herunterregulierter Gene erfolgen. (Dies kann z.B. durch Expression einer oder mehrerer kompensatorisch differentiell herunterregulierter Gene mit geeigneten Expressionsvektoren erfolgen.) Auf diese Weise kann ein dem Organismus eigener, durch das exprimierte Gen herbeigeführter Phänotyp, der durch die kompensatorisch differentielle Regulation eines oder mehrer Gene unterbunden wurde, zu Tage gebracht werden (vorzugsweise die Wachstumshemmung, insbesondere bei vielzelligen Organismen kommen hier aber auch andere Phänotypen in Frage).

Eine weitere Möglichkeit ist auch die Markierung eines oder mehrerer Gene, die kompensatorisch hochreguliert sind, mittels eines geeigneten Markers / Tags (der z.B. an das Genprodukt gekoppelt ist) oder mittels eines Reporters, der unter der Kontrolle des Enhancers und/oder Promotors des kompensatorisch hochregulierten Gens steht und in den Organismus eingeschleust wird. Geeignete Reporter sind dem zuständigen Fachmann bekannt, hier eignen sich insbesondere alle Arten von selbstieuchtenden Proteinen (z.B. GFP, BFP etc.), aber auch andere Reporter, mit denen ein detektierbares Signal erzeugt werden kann (z.B. Luziferase, β-Galaktosidase) sowie Wachstumsmarker für auxotrophe Stämme wie z.B. HIS3, URA3, LEU2, TRP1, und Antibiotika-Resistenz Gene wie z.B. für Kanamycin bzw. G418. Auch andere Arten der Phänotypisierung sind denkbar.

Im Anschluß an die Phänotypisierung ist es zweckmäßig, den Erfolg der Phänotypisierung durch geeignete Methoden (z.B. Messung der Proliferationsrate, Zeilzählung oder Bestimmung von Größe oder Morphologie, etc. und Vergleich mit dem Phänotyp bei nicht erfolgender heterologer Expression) zu überprüfen.

Gemäß einer bevorzugten Durchführungsform des erfindungsgemäßen Verfahrens erfolgt die Phänotypisierung durch Deletion, Mutagenese oder Überexpression mindestens eines kompensatorischen Gens.

Gemäß einer bevorzugten Ausführungsform erfolgt die Phänotypisierung durch Minderung! Aufhebung der kompensatorisch differentiellen Expression oder durch Markierung mindestens eines kompersatorischen Genes.

Die Expression des Proteins oder Proteinfragments kann dabei zur kompensatorischen Hoch- als auch Herunterregulation mindestens einer organismeneigenen Gens, aber auch dazu führen, dass eines oder mehrere Gene hoch-, eine oder mehrere andere herunterreguliert werden.

Besonders zweckmäßig Ist es auch, wenn die Expression des Proteins oder-Fragmentes induzierbar ist. Geeignete Systeme sind dem zuständigen Fachmann bekannt, so eignen sich beispielsweise Galaktose- oder Kupfer-regulierte Promotoren oder -Fragmentes induzierbar ist. Geeignete Systeme sind dem zuständigen Fachmann bekannt, so eignen sich beispielsweise Galaktos9-/ oder Kupfer-regulierte Promotoren, das Tet-On Tet-Off System, etc. Dabei kann entweder die Expression eines dem Organismus fremden oder eigenen Gens induzierbar angeschaltet werden (Induzierbarer Knock-In) oder die Expression eines dem Organismus eigenen Gens wird induzierbar vermindert oder ganz ausgeschaltet (induzierbarer Knock-Out).

Hierbei umfasst die gentechnische Veränderung zweckmäßigerweise die Einschleusung eines Vektors, der die induzierbare Expression des Proteins oder Proteinfragmentes ermöglicht, vorzugsweise eines mit Galactose- (GAL I/GAL 10) oder Kupfer- (CUPI) regulierten Promotors.

Tetracyclin induzierbaren Vektors oder von gewebsspezifisch induzierbare Promotoren wie z.B. hsp16-2, unc-119, unc-54, mec-7, oder myo-3 in C. elegans. Gemäß der vorliegenden Erfindung ist der Organismus eine Hefezelle, vorzugsweise eine Hefezelle vom Stamm S. cerevisiae.

Die Analyse der veränderten Genexpression wird bevorzugt durch DNA/RNA-Profiling mit Hilfe von cDNA oder Oligonukleotid-Microrrays durchgeführt. Alternativ erfolgt die Analyse mit Hilfe von Proteinarrays.

Bei einer vorteilhaften Ausgestaltung des Verfahrens erfolgt die Phänotypisierung durch Minderung oder Aufhebung der kompensatorisch differentiellen Regulation. Ist das kompensatorische Gen stärker exprimiert als in Kontrollorganismen, erfolgt die Minderung oder Aufhebung durch vollständige oder teilweise Inhibierung der verstärkten Expression. Vorzugsweise erfolgt dies durch Kreuzung mit einem Deletionsstamm und anschliessender Selektion der Doppelmutanten (eignet sich insbesondere bei Hefe als Organismus), durch genomischen Knock Out mit geeigneten Vektoren (diese sind dem Fachmann bekannt und ebenfalls sehr gut geeignet in Hefen, hier vor allem Saccharomyces cerevisiae), durch Mutagenese durch Strahlung und/oder mutagene Substanzen oder durch Einschleusung von antisense- Vektoren o.ä., die die Proteinproduktion des betreffenden Gens inhibieren. Hierbei ist es besonders vorteilhaft, wenn der Knock Out des kompensatorischen Gens den Knock In eines Reportergens wie z.B. β-Galaktoseidase, Luziferase oder Wachstumsmarker wie HIS3, ADE2, URA3, oder Resistenzmarker wie z.B. für Kanamycin umfasst. Das Reportergen kann dann im nachfolgenden Assay als Signal genutzt werden, die Wirksamkeit der zu testenden Wirksubstanzen zu detektieren und zu quantifizieren. Hierbei erfolgt vorzugsweise ein Austausch mindestens eines Teiles der kodierenden Sequenz des differentiell regulierten Gena gegen die kodierende Sequenz (umfasst auch Teile dieser Sequenz, die ausreichen, detektierbar zu sein) eines Reportergens (z.B. Luziferase, β-Galaktosldase etc). Ist das kompensatorische Gen weniger stark exprimiert als im Kontrollorganismus, erfolgt die Minderung oder Aufhebung durch Verstärkung der Expression, vorzugsweise durch Einkreuzung, Einschleusung eines episomalen oder eines anderen selektionsfähigen Expressionsvektors oder durch genomischen Knock-In (vorstehende Methoden eignen sich besonders gut für die Verwendung von Hefe als Organismus). Vorzugsweise führt die Minderung oder Aufhebung der kompensatorisch differentiellen Regulation zu einer Wachstumsinhibierung des gentechnisch veränderten Organismus, andere Phänotypen können aber ebenso vorteilhaft sein.

Die Beschreibung offenbart einen gentechnisch veränderten, phänotypisierten Organismus, der durch das erfindungsgemäße Verfahren erzeugt wurde.

Insbesondere offenbart die Beschreibung einen gentechnisch veränderten Organismus mit gentechnisch veränderter Expression mindestens eines eigenen oder fremden Gens, die zur kompensatorisch differentiellen Regulation mindestens eines anderen, dem Organismus eigenen Gens führt und so vorzugsweise das Auftreten eines aus Auswertbaren/detektierbaren/nutzbaren Phänotyps unterbindet oder hemmt, und mit durch Minderung/Aufhebung der kompensatorisch differentiellen Expression des Genes oder durch Markierung des kompensatorischen Genproduktes herbeigeführtem Phänotyp.

Die Beschreibung offenbart weiterhin die Verwendung eines erfindungsgemäß hergestellten gentechnisch veränderten Organismus zum Screening nach Substanzen mit einer Wirkung auf die Funktion des Proteins oder Proteinfragmentes sowie ein Verfahren zur Identifizierung von Substanzen mit einer Wirkung auf die Funktion des Proteins oder Proteinfragmentes.

Gemäß eines weiteren Aspektes offenbart die Beschreibung ebenso einen Assay zum Wirksubstanzscreening mit einem phänotypisierten Organismus durch Feststellung des Phänotyps (z.B. einer Wachstumsinhibierung infolge induzierter heterologer Überexpression eines Proteins), Inkontaktbringen der zu testenden Substanz mit dem Organismus und Beobachten einer möglichen Veränderung des Phänotyps, vorzugsweise dessen zumindest teilweisen Rückgang zu Verhalten bzw. Morphologie des Wildtyp-Organismus (also zumindest teilweiser Wiederherstellung des Phänotyps des Ausgangsorganismus, z.B. Aufhebung der Wachstumsinhibierung).

Die Erfindung wird nachfolgend anhand von Beispielen näher erläutert.

Beispiel1: Entwicklung einer Plattformtechnologie zur Identifizierung von Wirksubstanzen, die sich auf die Aktivität von Kinasen auswirken, auf Basis von Hefe als Organismus. Der herbeigeführte Phänotyp ist in diesem Fall die Wachstumshemmung der Hefen. Das Testprinzip beruht somit auf der Wachstumshemmung von Hefen, die als lebendes ,Reagenzglas" verwendet werden.

Unter Wachstumshemmung versteht man hier beispielsweise einen Zellzyklusarrest oder die Lyse der betroffenen Zellen. Hefen werden verwendet, da sie sich aufgrund ihrer genetischen Manipulierbarkeit ideal eignen. Humane (oder andere exogene) Kinasen werden in der Hefe unter der Kontrolle eines Galaktose-induzierbaren Promoters (GAL1/10) überexprimiert. Die Transformation und Kultivierung der Hefen erfolgt dabei nach Standardmethoden. Als Vektor werden z.B. Vektoren der Reihe p41 x-GAL1 oder p42x-GAL11 verwendet.

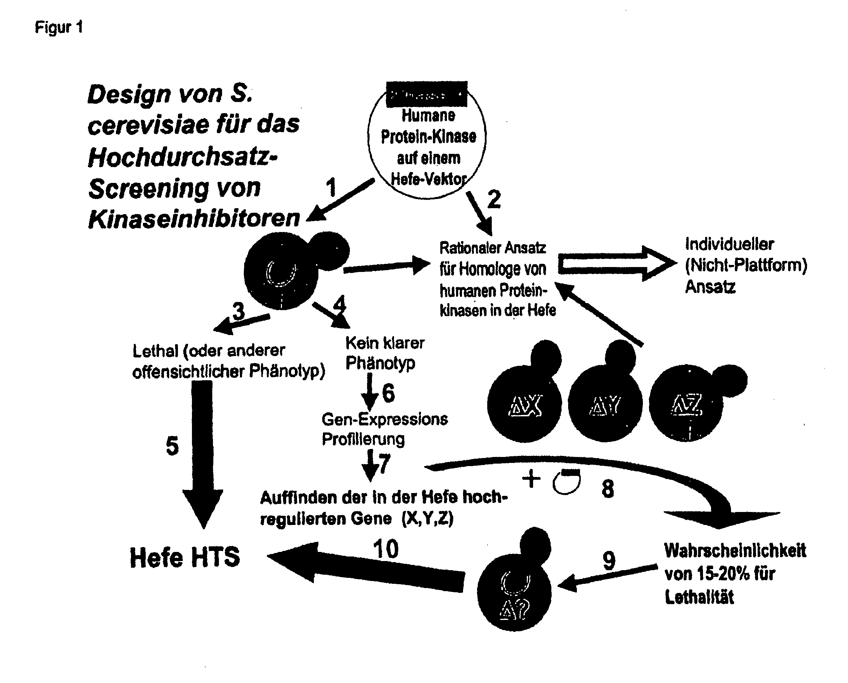

In ca. 30% aller zu testenden Kinasen wird die Überexpression bereits zur Wachstumshemmung in Hefe führen (Tugendreich et al. (2001)). Dieses Vorgehen wird in der Figur 1 mit den Schritten 1,3,5 dokumentiert. Kinasen, deren Überexpression zur Wachstumshemmung führt, werden in einen geeigneten Hefestamm integriert und anschließend ins Hochdurchsatzscreening (HTS) überführt. Hefestämme vom Stammhintergrund "MAT**a** his3□1 leu2□0 met15□0 ura3□0" (BY4741 von EUROSCARF) werden in diesem Beispiel verwendet.

Während der Assayentwicklung für das HTS werden die Bedingungen optimiert, indem verschiedene "Drug Transporter"-Deletionsmutanten im oben beschriebenen Stammhintergrund getestet werden. Für alle in diesem Beispiel zu testenden Proteinkinasen werden die Stämme mit den folgenden Deletions-Kombinationen getestet: 1. YRWS21 (MATa pdr1□::KanMX pdr3□::KanMX his3□1 leu2□0 met15□0 lys2□0 ura3□0) 2. YRWS39 (MAT**a** pdr5□::KanMX yor1□::KanMX his3□1 leu2□0 MET15 lys2□0 ura3□0) 3. YRWS14 (MAT**a** pdr5□::KanMX snq2□::KanMX his3□1 leu2□0 MET15 lys2□0 ura3□0)4. YRWS13 (MAT**a** snq2□::KanMX yor1□::KanMX his3□1 leu2□0 MET15 lys2□0 ura3□0) 5. YRWS44 (MAT**a** pdr5□::KanMX snq2□::KanMX yor1□::KanMX his3□1 leu2□0 met15□0 lys2□0 ura3□0).

Im Hochdurchsatzscreening kann dann nach biologischen und chemischen Molekülen gesucht werden, die die Wachstumshemmung mindern oder aufheben - d.h., die zum Wachsen der Hefekulturen führen. Alle bislang beschriebenen Techniken sind dem zuständigen Fachmann bekannt.

Wie oben beschrieben, verursachen ca. 30% aller exogenen Kinasen Wachstumshemmung in der Hefe. Daher verursachen ca. 70% aller überexprimierten

Kinasen keine oder nur geringe Wachstumshemmung. Um das Prinzip der Wachstumshemmung der Hefe als Plattformtechnik für das Compoundscreening von allen Proteinkinasen zu nutzen, müssen auch die verbleibenden 70% der Proteinkinasen eine Wachstumshemmung hervorrufen. Dazu bedarf es der vorliegenden Erfindung.

Die gewünschten Proteinkinasen werden in einen Hefe-Expressionsvektor der Wahl, in diesem Beispiel p413 GAL1 (D. Mumberg et al. (1994) in Volllänge und mit einem C-terminalen Tag, z.B. MYC-Tag, kloniert. Nach Transformation mit der Lithium-Azetat-Methode nach Standardprotokoll (s. Methods in Yeast Genetics) und Kultivierung In einem geeigneten Medium wird die Überexpression der exogenen Kinasen in der Hefe durch Zugabe von Galaktose nach Standardprotokoll (20 g/ml Medium) für 4 bis 6 Stunden bei 30°C induziert. Die Expression der Kinasen wird durch Immunoblots nach Standardprotokoll mit Hilfe von Antikörpern gegen den gewählten Tag (z.B: anti-MYC: AB1364 (Chemikon) oder M5546 (Sigma); anti-HA: HA-11-A (Biotrend) oder 55138 (ICN)) überprüft.

Nach dem immunologischen Expressionsnachweis in der Hefe werden Veränderungen der Genexpression - ausgelöst durch die Expression der exogenen Kinase - in der Hefe (die kompensatorisch differentielle Regulation) mit Hilfe von DNA-Microarrays untersucht DNA-Microarrays sind Trägermaterialen, an weiche spezifische Oligonukleotid chemisch gekoppelt sind. Die einzelnen Oligonukleotide repräsentieren hier individuelle Gene. DNA-Microarrays werden als Werkzeuge eingesetzt, die das momentane Expressionsmuster des gesamten Genoms der Hefe abdecken können. Für solch ein Experiment werden Kinase-transformierte Hefen mit mock-transformierten (leeres Plasmid) Hefen als Kontrolle verglichen. Aus beiden Stämmen wird die Gesamt-RNA mit Standardmethoden präpariert. Die RNA wird dann mit den Chip-gekoppelten Oligonukleotiden (auf den Microarrays) bei 45°C für 16 h hybridisiert. Der direkte Vergleich der Kinase-transformierten Hefe-RNA mit der mock-transformierten Hefe-RNA deckt Hefegene auf, die durch eine überexprimierte Proteinkinase kompensatorisch differentiell reguliert werden. Untersuchungen der Erfinder haben gezeigt, dass durch einen genetischen Eingriff, z.B. bei der Überexpression einer exogenen Proteinkinase, eine bestimmte Anzahl an RNAs für Hefegene hochreguliert und eine bestimmte Anzahl herunterreguliert werden (Tabelle 1). Das wurde am Beispiel der humanen Kinase PAK1 durchgeführt.

Tabelle 1: 2 Gene werden hochreguliert, 11 Gene werden herunterreguliert. Ferner konnten die Erfinder erstmals zeigen, dass viele der hochregulierten Gene aus kompensatorischen Gründen hochreguliert werden. In diesem Fall wurde ein S. cerevisiae-Wildtypstamm (W303-1a (Stammhintergrund oder Bezugsquelle)) verglichen mit Stamm, der eine Deletion im *Saccharomyces cerevisiae* Gen cla4 (□*cla4*) (YEL252) hat. Bis auf die Deletion In dem Gen für COLA4 sind beide Stämme isogen, d.h. identisch. Beim direkten Vergleich der RNA-Präparationen aus den zwei verschiedenen Stämmen (W303-1a und YEL252) tauchten 110 verschiedene RNAs aus dem Hefegenom als hochreguliert auf (Tabelle 2).

Tabelle 2: 56 Gene wurden herunterreguliert (Daten nicht gezeigt). Eine Erhöhung der RNA-Kopienzahl für bestimmte Gene könnte dabei möglicherweise aus kompensatorischen Gründen auftreten. Kompensatorisch bedeutet in diesem konkreten Beispiel, dass der durch die Deletion des CLA4-Gens verursachte Defekt im gentechnisch veränderten Stamm durch die vermehrte Expression von Genen abgeschwächt werden soll, die die Funktion von CLA4 ganz oder teilweise übernehmen können. Um diese These zu beweisen, wurden einige der hochregulierten Gene für weiterführende Experimente ausgewählt (siehe "2. Deletion" in Tabelle 3).

Tabelle 3: Dazu wurden MAT□-Hefestämme ausgesucht (können z.B von EUROSCARF oder Research Genetics bezogen werden), die Deletionen in den jeweils hochregulierten Genen tragen. Die Deletionen sind mit Markergenen gekennzeichnet, d.h. Markergene z.B. für eine Antibiotika-Resistenz oder für notwendige Wachstumsfaktoren wie z.B bestimmte Aminosäuren sind in das jeweilige Hefegenom integriert. Die ausgesuchten Deletionsstämme wurden nach hefegenetischen Standardmethoden (Methods in Yeast Genetics: A Cold Spring Harbor Course Manual (1994)) mit dem CLA4-Deletionsstamm (YEL252, MATa) gekreuzt.

Nach der Kreuzung wurde auf diploide Hefen selektiert. Diese wurden zur Sporenbildung veranlasst. Dabei entstehen aus einer diploiden Hefezelle 4 haploide Sporen, die zur Keimung In 4 haploide Hefeklone aufgeteilt werden können. Demnach kommt es zur Neuverteilung der Gene aus dem diploiden Stamm. In 25% aller Fälle werden die 2 Deletionen der unterschiedlichen Ausgangsstämme in einem neuen haploiden Klon vereint sein. Das kann anhand der verschiedenen Selektivmarker einfach verfolgt werden.

Mit dieser Standardmethode wurde versucht 13 verschiedene Doppeldeletionen herzustellen. In nur 10 Fällen waren die Doppeldeletionen lebensfähig, In 3 Fällen kam es nie zu der Doppeldeletion (Tabelle 3 "lethal"). In allen 3 Fällen wurden 40 Ascl getestet. Daher ist klar, dass die Kombination beider Deletionen zum Absterben der betroffenen Spore führt. Sie sind also synthetisch lethal. Es konnte gezeigt werden, dass in allen 13 Fällen die Doppeldeletionen entweder synthetisch lethal waren oder anderweitige synthetische Phänotypen gezeigt haben (Tabelle 3). Diese Untersuchung bestätigt die These, dass die betroffenen Gene hochreguliert wurden, um Defekte, ausgelöst durch das Fehlen von CLA4, zu kompensieren. Für die Erfindung ist wichtig, dass in den untersuchten Fällen (13 Doppeldeletionen) 3 Kombinationen und damit 23% aller möglichen Doppeldeletionen synthetische Lethalität zeigten (Tabelle 3).

Im Experiment mit dem □cla4-Stamm wurden 110 Gene hochreguliert (Tabelle 2). Auf die gleiche Weise wurden im oben beschriebenen Ansatz durch die Überexpression von humanem PAK1 die mRNAs für 2 Gene hochreguliert (Tabelle 1). Folglich werden auch diese Gene aus kompensatorischen Gründen hochreguliert. Aufgrund der geringen Anzahl an hoch regulierten Genen und der damit verbundenen niedrigen Erfolgsrate für synthetisch lethale Kombinationen, verzichteten wir auf das Folgeexperiment, Stämme zu identifizieren, die in der Kombination aus Deletionen in den hochregulierten Genen (mit YMR096W oder HIS3 aus Tabelle 1) und der Expression von humanem PAK1 einen synthetisch lethalen Phänotyp zeigten. Vielmehr wurde eine hyperaktive Mutante von humanem PAK1 hergestellt, nämlich humanes PAK1 DCRIB. Diese Mutante wurde wieder mit Standardmethoden in Hefe transformiert. Aufgrund der hohen Kinaseaktivität löste dieses Protein Wachstumshemmung in der Hefe aus. Ein geeigneter Stamm zum Testen für niedermolekulare Substanzen war identifiziert. Das Ziel war erreicht. Trotzdem wurde auch für diesen Fall ein differentielles Expressionsprofil mit den DNA-Microarrays aufgenommen, um die Validität der Erfindung zu untermauem (Tabelle 4).

Tabelle 4: 55 verschiedene Hefegene wurden aufgrund der hohen Kinaseaktivität kompensatorisch hochreguliert, 3 Gene wurden herabreguliert (nicht gezeigt). Für den Fall, dass die hohe Aktivität der PAK1-Mutante nicht ausgereicht hätte, um Wachstumshemmung in der Hefe auszulösen, könnten nun Deletionsstämme für die hochregulierten Gene getestet werden. Die PAK1-Mutante müsste in den jeweiligen Deletionsstämmen exprimiert werden. Den Wert einer 23%-igen Erfolgschance auf einen synthetischen Phänotyp zugrundelegend, würden dann in ca. 13 Hefestämmen die Expression der humanen PAK1-Mutante Wachstumshemmung hervorrufen. Damit wäre ein Stamm zum Testen von potenziellen Kinaseinhibitoren identifiziert In dem Fall des Testens von humanen Kinasen in der Hefe müssten die Ausgangsstämme nicht gekreuzt werden, da die humanen Kinasen Galaktoseabhängig von einem Plasmid exprimiert wird. Dieses Plasmid muss nur in den jeweiligen Deletionsstamm transformiert und die Expression der Kinase induziert werden. In 23% aller Fälle der zu testenden Stämme wird Wachstumshemmung (Lethalität) beobachten werden können. Die wachstumsgehemmten Stämme können aufgrund der jeweiligen Deletionen die Expression der plasmidkodierten Proteinkinase nicht mehr kompensieren. Damit können diese Systeme ins HTS überführt werden. Sollte eine Überexpression von bestimmten Wildtyp-Kinasen in Kombination mit dem DNA-Microarray-Experiment nicht ausreichen (wie oben für Wildtyp-PAK1 beschrieben, siehe Tabelle 2), um Wachstumshemmung hervorzurufen, dann werden Mutanten der jeweiligen Kinase hergestellt und anstelle der Wildtypkinasen eingesetzt (auch für die Genexprossionsexperimente mit den DNA-Microarrays). Diese Mutanten können nach dem Prinzip der zufälligen Mutagenese hergestellt werden, mit dem Ziel hyperaktive Mutanten zu gewinnen. Zur Mutagenese werden die Kinasekonstrukte mit einem C-terminalen Tag nach der Methode von Tugendreich et al. (2001) verwendet. Es wurde somit durch Arbeiten der Erfinder erstmals und überraschend gezeigt, dass die Deletion von kompensatorischen Genen zur Wachstumshemmung führen kann, verbunden mit der Erkenntnis einen standardisierten Plattformassay für Proteinkinasen aufzubauen. In den aktuellen Experimenten wurde Wachstumshemmung mit einer Frequenz von 23% nachgewiesen. Die Deletionsstämme, die nach der Transformation mit der plasmidkodierten Proteinkinase Wachstumshemmung zeigen, können nun wie oben beschrieben durch Optimierung (Austesten der verschiedenen "Drug-Transporter-Knockouts") ins HTS überführt werden. In der Figur 1 ist die Erfindung beispielhaft an Hand der Punkte 1,4,6-10 dargestellt.

Außer durch Einkreuzung der Deletionen kompensatorischer Gene hätte deren Deletion auch durch andere Methoden, wie genomischen Knock Out der Kinase exprimierenden Hefe selbst erfolgen können. Bei Hefen ist jedoch die Ausschaltung kompensatorischer Gene durch Einkreuzen von Deletionen oder der genomische Knock Out aufgrund der Einfachheit der Vorgehensweise besonders vorteilhaft. Bei anderen Organismen können sich demgegenüber eher andere Methoden eignen. So ist im Beispiel von eukaryontischen Zelllinien, und im Falle von mehrzelligen Organismen, wie Drosophila oder C. Elegans eher die Anwendung von Antisense-Verfahren wie RNAi geeignet. Die Auswahl von jeweils für die einzelnen Organismen geeigneten Maßnahmen liegt im Bereich fachmännischen Könnens.

Der erfindungsgemäße Plattformassay ermöglicht das HTS aller Proteinkinasen (wie an Hand von humanem PAK1 beschrieben) In homogenen und daher kostengünstigen Assaysystemen. Das System ist auch zur Bestimmung von IC₅₀-Werten im Compoundscreening geeignet.

Die Genexpressionsexperimente führen, wie im Beispiel beschrieben, auch zur identifizierung von RNAs von Genen, die durch die Expression von exogenen Kinasen reprimiert werden. Die Promotoren dieser reprimierten Gene können im HTS als Reporter dienen. Dazu werden die Hefepromotoren an sogenannte Reportergene wie β-Galactosidase, Luciferase, Wachstumsmarker wie HIS3, URA3, LEU2, oder TRP1, etc. fusioniert. Diese Konstrukte werden in den Hefestamm für das HTS transformiert. Dort dienen sie als Wachstumsmarker für Verbindungen, welche die Wachstumshemmung in dem betroffenen Stamm aufheben.

Beispiel 2: Der Plattformassay kann auch als sogenanntes Multiplexsystem eingesetzt werden. Unter Multiplexsystem wird verstanden, daß verschiedene Proteine oder Proteinfragmente z.B. Kinasen im gleichen Assay zur gleichen Zeit in einem Ansatz getestet werden. Dazu werden zunächst die individuellen phänotypisierten Hefestämme konstruiert. Die exogenen Proteinkinasen werden mit Standardmethoden integriert (s.o.). Dann werden diese Hefestämme zu einer homogenen Kultur vermischt. Die Expression der Proteinkinasen in dem homogenen Hefestamm Gemisch führt zur Wachstumshemmung, da auch die Expression jeder einzelnen Kinase an sich im phäntypisierten Hefestamm Wachstumshemmung auslöst. Im HTS werden Verbindungen identifiziert, die zum Wachstum von mindestens einem Hefestamm führen. Nun gilt es, den Verbindungen die betroffene Kinase zuzuordnen. Das wird über die sogenannte "colony PCR" Methode (A.J.P. Brown and M. Tuite (1998)) erreicht. Dazu werden einige, wenige Mikroliter aus den wachsenden Hefekulturen nach Anleitung (A.J.P. Brown and M. Tuite (1998)) lysiert. Aus (dem Gemisch an) genomischer DNA (inklusive integrierten Proteinkinasen) wird/werden mit spezifischen Primern für die unterschiedlichen Proteinkinasen die betroffene(n)/gehemmte(n) Kinase(n) durch quantitative RT-PCR zweifelsfrei identifiziert. Somit können durch das Mischen von verschiedenen Hefestämmen zu gleichen Teilen, unterschiedliche Kinasen in einem einzigen Screen getestet werden. Der Vorteil ist eine enorme Kosten- und Zeitersparnis.

Diese Technologie ist nicht nur auf Proteinkinasen anwendbar, sondern auf alle Proteine oder Substanzen, die eine transkriptionelle Antwort in der Hefe auslösen. Dieser Plattformassay ermöglicht im Gegenstand zu Assays des Standes der Technik z.B. das HTS aller Proteinkinasen (nicht nur solcher, die bereits auf Anhieb bei heterologer Expression einen Phänotyp hervorbringen) in homogenen und daher kostengünstigen Assaysystemen. Das System ist auch zur Bestimmung von IC₅₀-Werten im Compoundscreening geeignet.

Diese Technologie ist nicht nur auf Proteinkinasen anwendbar, sondern auf alle Proteine oder Substanzen, die eine transkriptionelle Antwort in der Hefe auslösen. Methoden:

Für genetische Manipulationen wurden die Standardmethoden nach Sambrook et al. (1989) Molecular Cloning: A Laboratory Manual. Second edition. Cold Spring Harbor Laboratory Press, Cold Spring Harbor, NY. 545 pp. eingesetzt.

Wachstumsbedingungen, Kreuzungsbedingungen und genetische Manipulationen an Hefen (Saccharomyces cerevisiae) wurden gemäß Guthrie, C. and G.R. Fink (1991) Guide to Yeast Genetics and Molecular Biology, Volume 194, J.N. Abelson and M.I. Simon, eds. (San Diego, CA: Academic Press Inc.) durchgeführt. Die Affymetrix-Experimente ("gene expression analysis) wurden exakt nach Klebl et al. (2001) Biochem. Biophys. Res. Commun. 286, 714-720 durchgeführt.

### Literatur:

Brown, A.J.P. and M. Tuite (1998). PCR-Based Gene Targeting in Saccharomyces cerevisiae. Methods Microbiol. 26, 67-81.
Methods in Yeast Genetics; A Cold Spring Harbor Course Manual; 1994 Edition; Kaiser, C., Michaelis, S., and A. Mitchell; Cold Spring Harbor Laboratory Press.
Mumberg, D., Müller, R. and M. Funk (1994). Regulatable promoters of Saccharomyces cerevisiae: comparison of transcriptional activity and their use for heterologous expression. Nucl. Acids Res. 22, 5767-5768.
Tugendreich, S., Perkins, E., Couto, J., Barthmaier, P., Sun, D., Tang, S., Tulac, S., Nguyen, A., Yeh, E., Mays, A., Wallace, E., Lila, T., Shivak, D., Prichard, M., Andrejka, L, Kim, R. and T. Melese (2001). A streamlined process to phenotypically profile heterologous cDNAs in parallel using yeast cell-based assays. Genome Res. 11, 1899-1912.

**Tabelle 1:**

| **2 Gene sind hochreguliert im ste20Δ Stamm YEL206, der hPAK1 exprimiert** | | |
|---|---|---|
| **Anmerkungen** | **Genfunktion** | **x-fach hoch reguliert** |
| YMR096W | Stationärphasenprotein | 2.15 |
| HIS3 | Imidazolglycerolphosphat Dehydratase; 7. Schritt der Histidin Biosynthese | 6.77 |

| **11 Gene sind herunterreguliert im ste20Δ Stamm YEL206, der hPAK1 exprimiert** | | |
|---|---|---|
| **Anmerkungen** | **Genfunktion** | **x-fach herunter reguliert** |
| STE20 | Serine/Threonine Proteinkinase des Pheromonresponse Signaltransduktionsweges | 47.62 |
| FRE7 | Protein mit schwacher Ähnlichkeit zu Fre1p und Fre2p, involviert in den Eisentransport | 11.70 |
| MFA1 | Matingpheromon a-Faktor, exportiert aus der Zelle durch Ste6p | 3.70 |
| YLR042C | Unbekannt | 3.27 |
| GPH1 | Glykogenphosphorylase, setzt α-D-Glukose-1-Phosphat frei | 2.63 |
| FRE1 | Eisen- und Kupferreductase, wirkt auf Fe2+ Ionen Chelate | 2.55 |
| YHR087W | Unbekannt | 2.31 |
| CWP1 | Mannoprotein der Zellwand; Mitglied der PAU1 Familie | 2.27 |
| YJL217W | Unbekannt | 2.25 |
| CTR1 | Kupfer Transportprotein; benötigt für hochaffine Aufnahme von Kupfer Ionen; | 2.17 |
| FET4 | Niedrigaffines Fe(II) Transportprotein | 2.00 |

**Tabelle 2:**

| **110 Gene sind hochreguliert im cla4□ Stamm YEL252** | | | |
|---|---|---|---|
| | **Anmerkungen** | **Genfunktion** | **x-fach hoch reguliert** |
| **Zellwand Aufrecht erhaltung** | FKS2 | Bestandteil der 0-1,3-Glucansynthase, funktioniert wahrscheinlich als alternative Untereinheit zu Fks1p (88% identisch); 55% identisch mit Fks3p; interagiert mit Rho1p; *fks*1Δ*fks2Δ* ist lethal | 6.81 |
| | ECM29 | Möglicherweise involviert in Zellwandstruktur oder Biosynthese | 3.13 |
| | SPI1 | Durch GPI Anker an die Zellwand gebunden; induziert durch Msn2/4p | 2.72 |
| | SBE22 | Notwendig für Wachstum der Buds; involviert in die Integrität der Zellwand | 2.08 |
| **Zellulärer stress** | HSP12 | 12 kDa Heat Shock Protein, induziert durch Hitze, osmotischen (HOG1-, PBS2-dependent) oder oxidativen Stress, stationäre Phase, HSF1, MSN2, YAP1; Chaperon (Mitglied der Hydrophilin Familie); 5 STREs | 6.55 |
| | HSP26 | Heat Shock Protein,induziert durch Osmostress, HSF1, MSN2, Hitze, H₂O₂; 29% identisch mit Hsp42p; Chaperon; 4 STREs | 4.76 |
| | HSP82 | Heat Shock Protein, 97% identisch mit Hsc82p, ähnlich dem Säuger HSP90 (komplementierbar durch humanes HSP90); Chaperon; induziert durch HSF1, SKN7, YAP1, H₂O₂; hat ATPase Aktivität; z.T. reguliert durch den HOG1 Signalweg, bindet an Ste11p; HSP90 Aktivität wird durch Sch9p moduliert | 2.67 |
| | GPX2 | Glutathionperoxidase, induziert durch YAP1 & Oxidantien | 2.64 |
| | SKN7 | Transkriptionsfaktor involviert in die Antwort auf oxidativen Stress (H₂O₂) & G1 Zellzykluskontrolle (Auftreten der Buds); interagiert mit Rho1p, Mbp1p, Cdc42p & genetisch mit PKC1; benätigt für das N₂-Entzug-induzierte pseudphyphale Wachstum; Kooperiert mit Yap1p bei der Genexpressionsinduktion; nicht involviert in den Heat Shock; Wirkt ggf im HOG1 Signalweg mit; Teil eines Zweikomponentensystems; Transkriptionsaktivierung stimuliert durch Skn7p ist abhängig vom Ras/PKA Signalweg | 2.60 |
| | SOD2 | Mitochondriale Mn2+ Superoxidedismutase, induziert durch HAP1,2,3,4,5 & reprimiert durch cAMP (RAS2); transkriptionelle Antwort auf H₂O₂ is Yap1p- & Skn7p-abhängig; induziert durch Msn2/4p | 2.57 |
| | ICT1 | k.o. höhere Widerstandsfähigkeit gegenüber Cu2+ als Wildtyp; mitochondriale Energie Transfer Signatur | 2.41 |
| | CYP2 | Mitglied der Cyclophilin Familie, Heat Shock Protein, Isomerase, Chaperon | 2.37 |
| | HSP42 | Heat Shock Protein, involviert in die Wiederherstellung des Zytoskeletts während leicheter Stresseinwirkung; induziert durch HOG1, MSN2/4, EtOH, H₂O₂; 3 STREs | 2.28 |
| | MSN4 | Transkriptionsfaktor, starke Ähnlichkeit zu Msn2p; Regulation der Trehalosekonzentration während Stress; 39 Gene abhängig von Msn2/4p für die Induktion bei diauxischem Shift und reprimiert durch cAMP: ALD3, GDH3, GLK1, HOR2, HSP104, HXK1, PGM2, SOD2, SSA3, SSA4, TKL2,TPS1, ARA,z.B. Ras2p kontrolliert die Stressresponse-Genexpression durch Msn2/4p & Yap1 p; TOR-Signaltransduktion kontrolliert die nukleäre Lokalisation von Nährstoff-regulierten Transkriptionsfaktoren | 2.15 |
| **Nukleotid Stoffwechsel** | ADE2 | Phosphoribosylaminoimidazol Karboxylase (AIR Dekarboxylase); weisse vs. rote Kolonien | 5.96 |
| | ADE17 | 5-Aminoimidazole-4-Karboxamid RibonuKleotid (AICAR) Transformylase/IMP Zyklohydrolase; weisse vs. rote Kolonien | 3.42 |
| | DCD1 | Deoxycyticylat Deaminase; k.o. hat gesteigerten dCTP Pool | 2.50 |
| **Transport kleiner Moleküle** | FRE7 | Involvid in uptake of copper and iron; weak similarity to Fre1p | 4.98 |
| | YHR048W | 29% identical to Ygr138p, Ypr156p, and 33% to Flr1p; MFS-MDR member | 4.20 |
| | PH089 | High-affinity Na+-dependent phosphate transporter; | 2.76 |
| | YGR138C | Member of the cluster I (family1) of the MFS-MDR; 89% identical to Ypr156p | 2.54 |
| | YER053C | MCF member | 2.40 |
| | TAF1 | Triacetylfuscerinine C transporter (MDR-MFS); 56%, 46%, 46% identical to Arn1p, Ycl073p, Ykr106p | 2.24 |
| | MUP3 | Low affinity amino acid permease (Met permease); APC family member | 2.16 |
| | ATM1 | ABC superfamily member, required for growth; may function in sensing iron; 43% identical to human ABC7 | 2.03 |
| **Carbohydrate metabolism** | GRE3 | NADPH-specific aldose reductase, induced by osmotic stress, MSN2/4, 0.1 M LiCl; 36%, 34%, 34% identical to Yjr096p, Gcy1p, Ypr1p; STREs and PDSEs; similar to human 305B protein (neonatal cholestatic hepatitis) | 3.61 |
| | GPH1 | Glycogen phosphorylase repressed by cAMP; stress-inducible | 3.49 |
| | GUT1 | Glycerol kinase, catalyzes conversion of glycerol to glycerol-3-phosphate, induced by ADR1, INO2, INO4, glycerol; strong similarity to human GK; activity is reduced during osmotic stress | 3.37 |
| | PCY1 | Pyruvate carboxylase I; converts pyruvate to oxalacetate for gluconeogenesis; 93%, 30%, 38% identical to Pyc2p, Hfa1p, Dur1,2p; similar to human PYC | 2.50 |
| | TSL1 | Component of trehalose-6-phosphate synthase/phosphatase complex; induced by STE12, STE7, TEC1, osmotic stress & repressed by cAMP, glucose; contains STREs | 2.40 |
| | GLK1 | Glucokinase specific for aldohexoses; 73%, 38%, 37% identical to Ydr516p, Hxk1 p, Hxk2p; induced by GCR1, HOG1, MSN2, MSN4 & repressed by cAMP, cold; protein increased upon H₂O₂, G1 phase | 2.09 |
| **Protein degradation** | YPS3 | GPI-anchored aspartyl protease (yapsin) at the plasma membrane; 45%, 36%, 47% identical to Mkc7p, Sst1p, Yps1p | 3.40 |
| | UBI4 | Ubiquitin polyprotein, mature ubiquitin is cleaved from polyubiquitin (Ubi4p) or from fusions with ribosomal proteins Rps31p, Rpl40Ap, Rpl40Bp; ribosomal heat shock protein & protein conjugation factor; 90% identical to Rpl40A/Bp and 100% to Rps31 p; induced HSF1, MSN2, starvation, heat shock; required for survival of cell stress; k.o. is hypersensitive to H₂O₂, N₂- and C₂-starvation; has STREs & HSEs | 3.27 |
| | VID24 | Required for vacuolar import and degradation of Fbp1p | 2.82 |
| | RPN10 | Non-ATPase component of the 26S proteasome complex, binds ubiquitin-lysozyme conjugates in vitro; C-terminus binds to ubiquitin | 2.46 |
| | BUL1 | Involved in ubiquitination pathway, binds to ubiquitin ligase | 2.12 |
| | AAP1 | Ala/Arg aminopeptidase, related to other Zn2+ metalloproteases & mammalian Zn2+ aminopeptidases | 2.00 |
| **DNA synthesis** | RIM1 | Transcription factor which binds ssDNA; required for replication in mitochondria | 3.27 |
| **Amino acid metabolism** | YMR250W | Similar to glutamate decarboxylase | 3.11 |
| | GDH2 | Glutamate DH, primary pathway to generate NH₄⁺ from glutamate, induced by rapamycin; gets phosphorylated in response to N₂ starvation (inactivation; PAK-dependent) | 2.83 |
| | GCV1 | Glycine decarboxylase T subunit, functions in pathway for Gly degradation | 2.31 |
| | CHA1 | Mitochondrial L-Ser/L-Thr deaminase, catalyzes conversion of Ser to pyruvate & Thr to □-ketobutyrate; induced by Ser, Thr, SIL1, CHA4 | 2.17 |
| **Signal transduction** | YGL179C | Ser/Thr protein kinase with similarity to Elm1p (31 %), Pak1 p (49%), Kin82p (30%), Gin4p (29%) | 3.10 |
| | KSP1 | Ser/Thr protein kinase that suppresses *prp20Δ* when overexpressed | 2.85 |
| | SLT2 | Ser/Thr protein kinase of the MAP kinase family involved in the cell wall integrity pathway, polarized growth, responseto nutrient availability, heat shock; interacts with Rlm1p, Swi4/6p, Mkk1/2p, Spa2p, Ptp2/3p, phosphorylates Swi4/6p & functions as regulator of the SBF complex; kinase activity induced by pheromone (requires Ste20p, but not Ste12p); kinase activity is cell cycle regulated | 2.77 |
| | STE20 | Ser/Thr protein kinase of pheromone response pathway, participates also in filamentous growth and STE vegetative growth pathways; | 2.25 |
| | YCK1 | CKI isoform, 77%, 50%, 41% identical to Yck2p, Yck3p, Hrr25p and 50-55% with human isoforms; gemaylgeranylated; yck1Δyck^{ts} displays hyperpolarized growth, hypersensitivity towards Zn²⁺ and multiple drugs, resistance to Mn²⁺ | 2.21 |
| | YHR046C | Myo-inositol-1 (or-4)-monophosphatase, participates in inositol cycle of Ca²⁺ signaling & inositol biosynthesis; similar to human MYOP (anti-manic, and - depressive actions of Li⁺) | 2.17 |
| | SCH9 | Ser/Thr protein kinase activated by cAMP; 46%, 44%, 42% identical to Ypk2p, Ypk1p, Tpk3p & 49% to human AKT1,2; controls FGM pathway; k.o. has modest defect in pseudohyphal growth and displays hyperinvasive growth | 2.17 |
| | PTP2 | PTPase involved in Hog1p and pheromone response pathways; interacts with Hog1p, Slt2p; induced by SLT2, YAP1, heat, osmotic stress; dephosphorylates Hog1p, Fus3p; posttranslationally regulated by Hog1p; 2 STREs | 2.01 |
| **Lipid, fatty acid & sterol metabolism** | PLB3 | Phospholipase B, releases GPI into the medium | 3.01 |
| | ERG7 | Lanosterol synthase (ergosterol biosynthesis), essential | 2.30 |
| **Membrane fusion** | YHR138C | Involved in vacuolar fusion with sequence similarity to Pbi2p | 2.81 |
| **Cell cycle control** | PCL5 | Cyclin that associates with Pho85p, belongs to Pcl1/2p subfamily | 2.73 |
| **polII transcription** | GAT2 | GATA Zn²⁺ finger transcription factor, required for expression of N₂ catabolite repression-sensitive genes | 2.73 |
| | HAP4 | Transcription factor, component of the Hap2/3/4/5p-complex involved in activation of CCAAT box-containing genes (SOD2, e.g.) | 2.48 |
| | STP4 | Transcription factor with strong homology to Stp1,2,3p; involved in tRNA splicing and branched-chain amino acid uptake | 2.17 |
| | SNF6 | Transcription factor, component of the SWI-SNF global transcription activator complex; acidic domains of Gcn4p, Swi5p, Hap4p interact directly with SWI-SNF complex | 2.13 |
| | SET1 | Transcription factor of the trithorax family of SET-domain-containing proteins, participates in control of transcription and chromosome structure; similar to human HRX Zn²⁺ finger protein | 2.04 |
| **Energy generation** | MDH2 | Cytosolic malate DH (glyoxylate cycle); induced by N₂ source limitation & repressed by cAMP, glucose; 3 STREs | 2.60 |
| **RNA processing/ modification** | RPP1 | Subunit of ribonuclease P & Rnase MRP ribonucleoprotein particles, needed for tRNA & 5.8S rRNA processing; 23% identical to hRpp30 | 2.49 |
| | PRP8 | U5 snRNA-associated splicing factor, essential RNA-binding protein; 62% identical to human PRP8; component of the spliceosome | 2.41 |
| | RRP4 | 3'-5'-exoribonuclease required for 3'-processing of ribosomal 5.8S rRNA; component of the nuclear & cytoplasmid forms of the 3'-5'-exosome complex; essential; induced in S-phase | 2.38 |
| | DBP8 | Similar to DEAD box family of RNA helicases | 2.33 |
| **Other metabolism** | YNL274C | Potential □-ketoisocaproate reductase, induced by YAP1, H₂O₂ | 2.26 |
| | DUR1,2 | Urea amidolyase, contains urea caroxylase & allophanate hydrolase activities; repressed by NH₄⁺ & induced by N₂ starvation, mating pheromone, Arg, rapamycin (N₂ utilization gene) | 2.21 |
| **Protein modification** | UBP5 | Ubiquitin-specific protease homologous to Doa4p & human Tre-2; member of rhodanese homology family | 2.17 |
| **Protein synthesis** | MSR1 | Mitochondrial arginyl-tRNA synthetase, 61% identical to Ydr341 p | 2.17 |
| **Vesicular transport** | SFB3 | Possible component of COPII vesicles, involved in transport of Pma1p from eR to Golgi; interacts with Sec23p | 2.17 |
| **Cytokinesis** | CDC12 | Essential part of the septin complex at the neck; required for pheromone-induced morphogenesis; septin assembly depends on Cla4p & Ste20p (Cdc42p, Cdc24p); mislocalized in yck2 | 2.09 |
| **Mating response** | SSF1 | Suppressor of sterile four; 94% identical to Ssf2p; ssf1□ssf2□ is lethal; multicopy suppressor of hsp90-loss-of-function mutation | 2.06 |
| **Unknown** | YHR214W | 100%, 77%, 74% identical to Yar066p, Yil169p, Yol155p | 9.88 |
| | YAR066W | 100%, 77%, 74% identical to Yhr214p, Yil169p, Yol155p | 7.59 |
| | RTA1 | Resistant to aminocholesterol; induced by TEC1, STE7, STE12 | 4.64 |
| | MSC1 | Functions in the meiotic homologous chromatid recombination pathway | 4.62 |
| | YHL021C | Induced by STE12, TEC1, STE7 | 4.35 |
| | YHR209W | Putative SAM-dependent methyltransferase | 4.26 |
| | COS8 | Protein family of conserved sequences | 3.74 |
| | YNR014W | 30% identical to Ymr206p; 4 putative STREs | 3.44 |
| | YIR042C | - | 3.37 |
| | YCL049C | - | 3.28 |
| | YHR087W | - | 3.19 |
| | YHR078W | 4 potential transmembrane segments | 3.00 |
| | TRA1 | Essential component of the Ada-Spt transcriptional regulatory complex (SAGA), SAGA-like complex, & NuA4 complex | 2.82 |
| | BTN2 | Elevated expression with yhc3Δ; 38% identical to human HOOK1 | 2.77 |
| | VAB36 | Vac8p-binding protein of 36 kDa; 2 putative STREs | 2.75 |
| | YFL063W | Similar to subtelomeric proteins | 2.68 |
| | YHR112C | Similar to cystathione □-synthase Str2p & other transulfuration enzymes, also similar to human CGL (cystathioninuria) | 2.56 |
| | YBL064C | Mitochondrial thiol peroxidase of the 1-Cys family; one of the 4 peroxidases in S.c.; uses thioredoxin as electron donor; induced upon oxidative stress; reduces H₂O₂ in the presence of DTT | 2.55 |
| | YSC83 | Induced mRNA levels during sporulation | 2.46 |
| | BOP1 | Bypass of PAM1 (PAM1 = multicopy suppressor of loss of PP2A) | 2.45 |
| | YHR045W | 5 potential transmembrane domains | 2.44 |
| | YHR033W | Induced by N₂ source limitation & repressed by cAMP | 2.42 |
| | YPR009W | Putative Zn²⁺-finger domain; 34% identical to Sut1p | 2.40 |
| | YLL064C | Member of the seripauperin family | 2.39 |
| | YPL137C | Similar to Mhp1p (27%), Yor227p (43%) | 2.39 |
| | YHR182W | - | 2.37 |
| | YDR222W | - | 2.37 |
| | YHR146W | Similar to pheromone adaption protein Mdg1p | 2.36 |
| | YMR184W | - | 2.36 |
| | YGL261C | Member of the seripauperin (PAU) family | 2.34 |
| | YHR083W | Essential | 2.32 |
| | YHR122W | Essential | 2.29 |
| | YOR227W | 43%, 25% identical to Ypl137p, Mhp1p | 2.27 |
| | YHR186C | WD40 domain; essential | 2.26 |
| | YHR073W | Similar to human oxysterol-binding protein; interacts with Spo12p | 2.20 |
| | YJL217W | - | 2.17 |
| | YHR192W | - | 2.11 |
| | YDL231C | Putative Zn²⁺ finger domain | 2.10 |
| | YDR391C | 57%, 41% identical to Yor013p, Yor012p | 2.05 |

**Tabelle 3:**

| Name des Stammes | 1. Deletion | 2. Deletion | Phänotyp |
|---|---|---|---|
| W303-1a | - | - | kein |
| YEL252-1a | cla4 | - | Zytokinese |
| YAS | cla4 | ptp2 | synthetisch |
| YAS | cla4 | glk1 | synthetisch |
| YAS | cla4 | msn4 | synthetisch |
| YAS | cla4 | ygl173 | synthetisch |
| YAS | cla4 | gut1 | synthetisch |
| YAS | cla4 | rta1 | geheilt |
| YAS | cla4 | skn7 | synthetisch |
| YAS | cla4 | pde2 | synthetisch |
| YAS | cla4 | yck1 | synthetisch, extrem langsames Wachstum |
| YAS | cla4 | sbe22 | synthetisch |
| YAS | cla4 | elm1 | lethal |
| YAS | cla4 | slt2 | lethal |
| YAS | cla4 | ste20 | lethal |

**Tabelle 4:**

| **55 Gene sind hochreguliert im ste20Δ Stamm YEL206, der hPAK1ΔCRIB exprimiert** | | |
|---|---|---|
| **Anmerkungen** | **Genfunktion** | **x-fach hoch reguliert** |
| PHO5 | Reprimierbare saure Phosphatase; benötigt Glykosylierung für Aktivität | 10.19 |
| ZRT1 | Hochaffines Zink Transportprotein; Mitglied der ZIP Familie | 10.12 |
| PHO11 | Sezernierte Saure Phosphatase | 7.67 |
| HSP30 | Heat shock Protein, lokalisiert in Plasmamembran | 6.30 |
| PHO12 | Sezemierte Saure Phosphatase | 5.80 |
| YIL057C | Unbekannt | 5.70 |
| YOL154W | Protein mit Ähnlichkeit zu Zink Metalloproteinasen | 5.24 |
| YPL274W | Hochaffine S-Adenosylmethionin Permease | 5.16 |
| CIT3 | Mitochondriale Zitratsynthase | 5.15 |
| RTA1 | Protein, das in den 7-Aminocholesterol Wiederstand involviert ist | 5.14 |
| YEL070W | Protein mit Ähnlichkeit zu E.coli D-Mannonat Oxidoreductase | 5.09 |
| YDL037C | Protein mit Ähnlichkeit zu Glucan 1,4-□-Glukosidase | 4.95 |
| YHR136C | Putativer Inhibitor des Pho80-Pho85p Cyclin-abhängignen Kinase Komplexes | 4.84 |
| LEE1 | Unbekannt | 4.59 |
| YMR303C | Alkohol Dehydrogenase II; oxidiert Ethanol zu Acetaldehyde | 4.07 |

## Patentansprüche

1. Verfahren zur Generierung eines gentechnisch veränderten eukaryontischen einzelligen Organismus für das Wirksubstanzscreening, bei dem:
a) ein Protein oder Proteinfragment in einem eukaryontischen einzelligen Organismus durch gentechnische Veränderung exprimiert wird,
b) das **dadurch** veränderte Genexpressionsmuster des eukaryontischen einzelligen Organismus mit Hilfe eines DNA- oder Protein-Microarrays analysiert wird,
c) Gene identifiziert werden, deren Expression oder Abschalten die Auswirkungen der Expression des Proteins oder Proteinfragmentes kompensieren, und
d) durch Minderung, Aufhebung oder Verstärkung der Expression dieser kompensatorischen Gene ein Phänotyp in dem gentechnisch veränderten eukaryontischen einzelligen Organismus hervorgerufen wird, wobei der Phänotyp die Wachstumshemmung des eukaryontischen einzelligen Organismus ist,
wobei der eukaryontische einzellige Organismus eine Hefezelle ist.

2. Verfahren nach Anspruch 1, **dadurch gekennzeichnet, dass** die gentechnische Veränderung die Expression mindestens eines dem Organismus eigenen und oder fremdem Proteins oder Proteinfragmentes bewirkt.

3. Verfahren nach einem der Ansprüche 1 oder 2, **dadurch gekennzeichnet, dass** die gentechnische Veränderung die Verminderung oder Ausschaltung der Expression mindestens eines dem Organismus eigenen Proteins bewirkt.

4. Verfahren nach einem der Ansprüche 1 bis 3, **dadurch gekennzeichnet, dass** die durch gentechnische Veränderung veränderte Expression des Proteins oder Proteinfragmentes induzierbar ist.

5. Verfahren nach Anspruch 4, **dadurch gekennzeichnet, dass** die gentechnische Veränderung die Einschleusung eines Vektors umfasst, der die induzierbare Expression des Proteins oder Proteinfragmentes ermöglicht.

6. Verfahren nach Anspruch 5, **dadurch gekennzeichnet, dass** der Vektor ein mit Galactose, Kupfer oder Tetracyclin induzierbarer Vektor ist.

7. Verfahren nach einem der Ansprüche 1 bis 6, **dadurch gekennzeichnet, dass** die gentechnische Veränderung einen Knock Out umfasst.

8. Verfahren nach einem der Ansprüche 1 bis 6, **dadurch gekennzeichnet, dass** das kompensatorische Gen gegenüber Kontrollorganismen verstärkt exprimiert wird und die Minderung oder Aufhebung durch zumindest teilweise Inhibierung der verstärkten Expression erfolgt.

9. Verfahren nach Anspruch 8, **dadurch gekennzeichnet, dass** die Inhibierung der verstärkten Expression des kompensatorischen Gens durch Knock Out durchgeführt wird.

10. Verfahren nach Anspruch 9, **dadurch gekennzeichnet, dass** der Knock Out des kompensatorischen Gens durch den Austausch mindestens eines Teiles der kodierenden Sequenz des kompensatorischen Gens gegen die kodierende Sequenz eines Reportergens oder Teile der Reportergensequenz, die ausreichen, detektierbar zu sein, erfolgt.

11. Verfahren nach Anspruch 9, **dadurch gekennzeichnet, dass** das kompensatorische Gen weniger stark exprimiert wird als in Kontrollorganismen und die Minderung oder Aufhebung durch Verstärkung seiner Expression erfolgt.

## Claims

1. A method for generating a genetically modified eukaryotic unicellular organism for drug screening, comprising
a) expressing a protein or protein fragment in a eukaryotic unicellular organism by means of genetic modification,
b) analyzing the thereby modified gene expression pattern of said eukaryotic unicellular organism with the aid of a DNA or protein microarray,
c) identifying genes whose expression or switching-off compensates for the effects of expression of said protein or protein fragment, and
d) a phenotype being produced in said genetically modified eukaryotic unicellular organism by reducing, eliminating or enhancing expression of these compensating genes, said phenotype being the inhibition of growth of said eukaryotic unicellular organism, with said eukaryotic unicellular organism being a yeast cell.

2. The method as claimed in claim 1, wherein the genetic modification causes expression of at least one protein or protein fragment which is endogenous to the organism and/or foreign.

3. The method as claimed in either of claims 1 and 2, wherein the genetic modification causes reduction or elimination of the expression of at least one protein endogenous to the organism.

4. The method as claimed in any of claims 1 to 3, wherein the expression, modified by genetic modification, of the protein or protein fragment is inducible.

5. The method as claimed in claim 4, wherein the genetic modification comprises introducing a vector which enables the protein or protein fragment to be inducibly expressed.

6. The method as claimed in claim 5, wherein the vector is a vector inducible with galactose, copper or tetracycline.

7. The method as claimed in any of claims 1 to 6, wherein the genetic modification comprises a knock out.

8. The method as claimed in any of claims 1 to 6, wherein the compensating gene undergoes enhanced expression to control organisms and the reduction or elimination is caused by at least partial inhibition of said enhanced expression.

9. The method as claimed in claim 8, wherein inhibition of said enhanced expression of the compensating gene is carried out by knock out.

10. The method as claimed in claim 9, wherein the knock out of the compensating gene is carried out by replacing at least part of the coding sequence of the compensating gene with the coding sequence of a reporter gene or parts of the reporter gene sequence which are sufficient to be detected.

11. The method as claimed in claim 9, wherein the compensating gene is less strongly expressed than in control organisms and the reduction or elimination is carried out by enhancing its expression.

## Revendications

1. Procédé de production d'un organisme unicellulaire eucaryote génétiquement modifié pour le criblage de principes actifs, dans lequel :
a) une protéine ou un fragment de protéine est exprimé, par modification génétique, dans un organisme unicellulaire eucaryote,
b) le modèle d'expression génique ainsi modifié de l'organisme unicellulaire eucaryote est analysé à l'aide d'un microréseau d'ADN ou de protéines,
c) des gènes sont identifiés, dont l'expression ou la désactivation compensent les effets de l'expression de la protéine ou du fragment de protéine, et
d) par diminution, suppression ou renforcement de l'expression de ces gènes compensateurs, un phénotype est créé dans l'organisme unicellulaire eucaryote génétiquement modifié, le phénotype étant l'inhibition de la croissance de l'organisme unicellulaire eucaryote, l'organisme unicellulaire eucaryote étant une cellule de levure.

2. Procédé selon la revendication 1, **caractérisé en ce que** la modification génétique provoque l'expression d'au moins une protéine ou un fragment de protéine, propre et/ou étranger à l'organisme.

3. Procédé selon l'une des revendications 1 ou 2, **caractérisé en ce que** la modification génétique provoque la diminution ou la suppression de l'expression d'au moins un produit propre à l'organisme.

4. Procédé selon l'une des revendications 1 à 3, **caractérisé en ce que** l'expression de la protéine ou du fragment de protéine, modifiée par modification génétique, est inductible.

5. Procédé selon la revendication 4, **caractérisé en ce que** la modification génétique comprend l'introduction d'un vecteur, qui permet l'expression inductible de la protéine ou du fragment de protéine.

6. Procédé selon la revendication 5, **caractérisé en ce que** le vecteur est un vecteur inductible par le galactose, le cuivre ou la tétracycline.

7. Procédé selon l'une des revendications 1 à 6, **caractérisé en ce que** la modification génétique comprend une invalidation génique (knock-out).

8. Procédé selon l'une des revendications 1 à 6, **caractérisé en ce que** le gène compensateur est exprimé d'une manière renforcée par rapport à des organisme témoins, et la diminution ou la suppression ont lieu grâce à une inhibition au moins partielle de l'expression renforcée.

9. Procédé selon la revendication 8, **caractérisé en ce que** l'inhibition de l'expression renforcée du gène compensateur est réalisée par invalidation génique.

10. Procédé selon la revendication 9, **caractérisé en ce que** l'invalidation génique du gène compensateur est réalisée par remplacement d'au moins une partie de la séquence codante du gène compensateur par la séquence codante du gène rapporteur ou par des parties de la séquence du gène rapporteur qui suffisent pour être détectables.

11. Procédé selon la revendication 9, **caractérisé en ce que** le gène compensateur est exprimé moins fortement que les organismes témoins, et la diminution ou la suppression a lieu par renforcement de son expression.
